# EUROPEAN PATENT APPLICATION

(11) **EP 1 727 305 A1**
(43) Date of publication of application: **29.11.2006**
(21) Application number: 05720247.5
(22) Date of filing: 08.03.2005
(51) Int. Cl.: H04B 11/00, A61B 6/00, A61B 5/055

(54) **DATA COMMUNICATION APPARATUS**

(30) Priority: 09.03.2004 JP 2004065936
(71) Applicant: Nemoto Kyorindo Co., Ltd., Tokyo 113-0033 (JP)
(72) Inventor: Ono, Seiichi, c/o Nemoto Kyorindo Co., Ltd., Bunkyo-ku, Tokyo 113-0033 (JP)
(74) Representative: Nargolwalla, Cyra
(86) International application number: PCT/JP2005/003975
(87) International publication number: WO 2005/086393

(57) **Abstract**

Upper transmitter 141 converts an electric signal transmitted from upper electronic device 101 into an ultrasonic signal and transmits the ultrasonic signal. First communication medium 146 transports the ultrasonic signal to lower receiver 142. Lower receiver 142 converts the received ultrasonic into an electric signal and transmits the electronic signal to lower electronic device 110. Thus, the electronic signal transmitted from upper electronic device 101 to lower electronic device 110 is modulated into the ultrasonic signal and then the ultrasonic signal is demodulated into the electronic signal, thereby the electronic signal cannot return to upper electronic device 101. Therefore, a data communication apparatus which can reliably regulate the direction of the transmitted electronic signal is provided.

## Description

### Technical Field

The present invention relates to a data communication apparatus which intervenes in communication of electric signals between an upper electronic device and a lower electronic device, and more particularly, to a data communication apparatus which intervenes in signal communication between an upper electronic device and a lower electronic device which are placed in different rooms separated by a wall.

### Background Art

Presently available diagnostic imaging apparatuses for capturing diagnostic images of patients include CT (Computed Tomography) scanners, MRI (Magnetic Resonance Imaging) apparatuses, PET (Positron Emission Tomography) apparatuses, ultrasonic diagnostic apparatuses and the like. CT angiography apparatuses, MRA (MR angiography) apparatuses and the like are currently used as medical apparatuses for capturing vascular images of patients.

Such diagnostic imaging apparatuses typically have a diagnostic imaging unit and an imaging control unit as separate components. The diagnostic imaging unit captures diagnostic images of patients and outputs them to the outside. The imaging control unit is manually operated by an operator to remotely control the diagnostic imaging unit to receive the diagnostic images therefrom for output as display observed by the operator.

In this manner, the diagnostic imaging unit is placed with the patient and the imaging control unit is placed with the operator. From a hygiene viewpoint, the diagnostic imaging unit and the imaging control unit are typically disposed in different rooms separated by a wall. In a facility such as a hospital where the diagnostic imaging apparatus is installed, a through hole is formed in a wall which separates rooms where the diagnostic imaging unit and the imaging control unit are placed, and a communication cable is inserted into the through hole to connect the diagnostic imaging unit with the imaging control unit, or communication cables from the diagnostic imaging unit and the imaging control unit are connected to a connector buried in the wall as a data communication apparatus.

When the abovementioned diagnostic imaging apparatus is used, a liquid such as a contrast medium or physiological saline may be injected into a patient. Chemical liquid injectors for automatically performing the injection have been put into practical use. Such a chemical liquid injector has a driving motor, a slider mechanism and the like, for example. A liquid syringe is removably mounted on the injector.

The liquid syringe includes a cylinder member and a piston member slidably inserted into the cylinder member. The cylinder member is filled with a contrast medium or physiological saline. When the liquid syringe is connected to a patient through an extension tube and set on a liquid injection mechanism, the chemical liquid injector individually holds the piston member and the cylinder member and relatively moves them with the liquid injection mechanism to inject the contrast medium or physiological saline from the liquid syringe into the patient.

When the diagnostic imaging apparatus and the chemical liquid injector described above are used, the diagnostic imaging unit and the chemical liquid injector are placed in a treatment room, and the imaging control unit is placed in an operation room which is separated from the treatment room by a wall, for example. An operator is located in each of the treatment room and the operation room. One of the two operators operates the chemical liquid injector and the other controls the diagnostic imaging apparatus.

One of the operators manually operates the chemical liquid injector in the treatment room to inject the contrast medium into a patient, while the other manually operates the imaging control unit in the operation room to cause the diagnostic imaging unit in the treatment room to capture diagnostic images.

Some of the chemical liquid injectors described above have an injection operation unit and an injection control unit which are formed as separate components and wire-connected. When such a chemical liquid injector is used, it is possible to place the injection operation unit in the treatment room and the injection control unit in the operation room, by way of example.

In this case, a through hole is formed in the wall which separates the treatment room and the operation room, and a communication cable is inserted into the through hole to connect the injection operation unit with the injection control unit, or communication cables from the injection operation unit and the injection control unit are connected to a connector buried in the wall as a data communication apparatus.

Various data communication apparatuses of the type described above intervening in data communication between the diagnostic imaging unit and the imaging control unit of the diagnostic imaging apparatus have been proposed (see, for example, patent documents 1 to 3 below).
Patent document 1: Japanese laid-open patent publication No. 07-178169;
Patent document 2: Japanese laid-open patent publication No. 07-204176;
Patent document 3: Japanese laid-open patent publication No. 2003-534859;

### Disclosure of the Invention

### Subject to Be solved by the Invention

The abovementioned communication cable includes a conducting wire for transmitting electric signals. The electric signals may be designed to transmit only in one direction through the conducting wire. However, the conducting wire can transmit electric signals bidirectionally, so that noise or the like may be generated in a signal receiver and then transmitted to a signal transmitter to cause a malfunction.

To prevent the malfunction, a rectifier circuit having diode is connected to the conducting wire for transmitting electric signals only in one direction. However, the rectifier cannot provide perfect rectification and may not prevent overvoltage or the like.

The present invention has been made in view of the abovementioned problems, and it is an object thereof to provide a data communication apparatus which can reliably regulate the direction of transmission of electric signals.

### Means to Solve the Subject

According to the first aspect of the present invention, a data communication apparatus for intervening in communication of an electric signal from an upper electronic device to a lower electronic device, comprises an ultrasonic transmitter, an ultrasonic receiver, and an ultrasonic communication medium. The ultrasonic transmitter converts the electric signal transmitted from the upper electronic device into an ultrasonic signal and transmits the ultrasonic signal. The ultrasonic receiver receives and converts the ultrasonic signal transmitted from the ultrasonic transmitter into an electric signal and transmits the electric signal to the lower electronic device. The ultrasonic communication medium transports the ultrasonic signal, and the ultrasonic transmitter and the ultrasonic receiver are placed in the ultrasonic communication medium such that they are separated from each other. Thus, the electric signal transmitted from the upper electronic device to the lower electronic device is subjected to modulation into the ultrasonic signal and then demodulation into the electric signal.

According to the second aspect of the present invention, a data communication apparatus for intervening in communication of an electric signal between an upper electronic device and a lower electronic device, comprises an upper transmitter, a lower receiver, a lower transmitter, an upper receiver, a first communication medium, and a second communication medium. The upper transmitter converts the electric signal transmitted from the upper electronic device into an ultrasonic signal and transmits the ultrasonic signal. The lower receiver receives and converts the ultrasonic signal transmitted from the upper transmitter into an electric signal and transmits the electric signal to the lower electronic device. The lower transmitter converts an electric signal transmitted from the lower electronic device into an ultrasonic signal and transmits the ultrasonic signal. The upper receiver receives and converts the ultrasonic signal transmitted from the lower transmitter into an electric signal and transmits the electric signal to the upper electronic device. The first communication medium transports the ultrasonic signal, and the upper transmitter and the lower receiver are placed in the first communication medium such that they are separated from each other. The second communication medium transports the ultrasonic signal, and the lower transmitter and the upper receiver are placed in the second communication medium such that they are separated from each other. Thus, the electric signal transmitted from the upper electronic device to the lower electronic device and the electric signal transmitted from the lower electronic device to the upper electronic device are subjected to modulation into the ultrasonic signals and then demodulation into the electric signals.

The upper electronic device and the lower electronic device referred to in the present invention mean any devices which at least provides output of an electric signal and at least receives input of an electric signal, respectively. In other words, the "upper" side means an electronic device which at least provides output of an electric signal, while the "lower" side means an electronic device which at least receives input of an electric signal. When a pair of electronic devices can transmit an electric signal bidirectionally, either of them can be defined as the upper or lower device.

Various means referred to in the present invention may be arranged to perform their functions, and may comprise dedicated hardware for performing a predetermined function, a data processing apparatus whose predetermined function is given by a computer program, a predetermined function performed in a data processing apparatus according to a computer program, or a combination thereof.

Various components referred to in the present invention do not need to be a separate entity. A plurality of means may be constructed as one member, a certain means may be part of another means, or a certain means may have a portion overlapping a portion of another means.

### Effect of the Invention

In the data communication apparatus of the present invention, since the electric signal transmitted between the upper electronic device and the lower electronic device is modulated into the ultrasonic signal and then demodulated into the electric signal, the direction of the transmitted electric signal can be reliably regulated.

### Brief Description of the Drawings

Fig. 1 is a block diagram showing the circuit structure of a chemical liquid injector according to an embodiment of the present invention.
Fig. 2 is a block diagram showing the circuit structure of an MRI apparatus which is an diagnostic imaging apparatus according to the embodiment of the present invention.
Fig. 3 is a schematic perspective view showing the layout of the MRI apparatus and the chemical liquid injector.
Fig. 4 is a perspective view showing the exterior appearance of the chemical liquid injector.
Fig. 5 is a perspective view showing how to mount a liquid syringe on an injection head of the chemical liquid injector.
Fig. 6 is a perspective view showing the main portions of a data communication member which is a data communication apparatus.
Fig. 7 is a perspective view showing the main portions of a data communication unit which is a data communication apparatus.
Fig. 8 is a schematic perspective view showing the layout of an MRI apparatus and a chemical liquid injector of a variation of the present invention. Description of Reference Numerals
   - 100, 500: CHEMICAL LIQUID INJECTOR
   - 101: INJECTION CONTROL UNIT SERVING AS UPPER ELECTRONIC DEVICE
   - 103: OPERATION PANEL SERVING AS INJECTION INPUT MEANS
   - 104: TOUCH PANEL SERVING AS INJECTION INPUT MEANS
   - 110: INJECTION HEAD SERVING AS LOWER ELECTRONIC DEVICE AND INJECTION OPERATION UNIT
   - 116: PISTON DRIVING MECHANISM SERVING AS LIQUID INJECTION MEANS
   - 121: COMMUNICATION I/F CORRESPONDING TO INJECTION RECEIVING MEANS
   - 134: COMMUNICATION I/F CORRESPONDING TO INJECTION TRANSMITTING MEANS
   - 140: DATA COMMUNICATION MEMBER SERVING AS DATA COMMUNICATION APPARATUS
   - 141: UPPER TRANSMITTER
   - 142: LOWER RECEIVER
   - 143: LOWER TRANSMITTER
   - 144: UPPER RECEIVER
   - 146: FIRST COMMUNICATION MEDIUM
   - 147: SECOND COMMUNICATION MEDIUM
   - 151: FIRST TUBE CHAMBER SERVING AS FIRST SEALING CASE
   - 152: SECOND TUBE CHAMBER SERVING AS SECOND SEALING CASE
   - 153: CONNECTING PORTION SERVING AS CASE PARALLELING MEANS
   - 200: MRI APPARATUS SERVING AS DIAGNOSTIC IMAGING APPARATUS
   - 201: DIAGNOSTIC IMAGING UNIT SERVING AS UPPER ELECTRONIC DEVICE
   - 210: IMAGING CONTROL UNIT SERVING AS UPPER ELECTRONIC DEVICE
   - 214: KEYBOARD SERVING AS IMAGING INPUT MEANS
   - 215: DISPLAY SREVING AS IMAGE DISPLAY MEANS
   - 216: COMMUNICATION I/F CORRESPONDING TO IMAGING TRANSMITTING MEANS AND IMAGE RECEIVING MEANS
   - 230: DATA COMMUNICATION UNIT SERVING AS DATA COMMUNICATION APPARATUS
   - 231: UPPER MDULATOR
   - 232: UPPER TRANSMITTER
   - 233: LOWER RECEIVER
   - 234: LOWER DEMODULATOR
   - 236: LOWER MODULATOR
   - 237: LOWER TRANSMITTER
   - 238: UPPER RECEIVER
   - 239: UPPER DEMODULATOR
   - 241: FIRST COMMUNICATION MEDIUM
   - 242: SECOND COMMUNICATION MEDIUM
   - 250: UNIT BODY SERVING AS CASE PARALLELING MEANS
   - 251: FIRST SEALING CASE
   - 252: SECOND SEALING CASE
   - 300: WALL

### Best Mode for Carrying the Invention

### (Configuration of Embodiment)

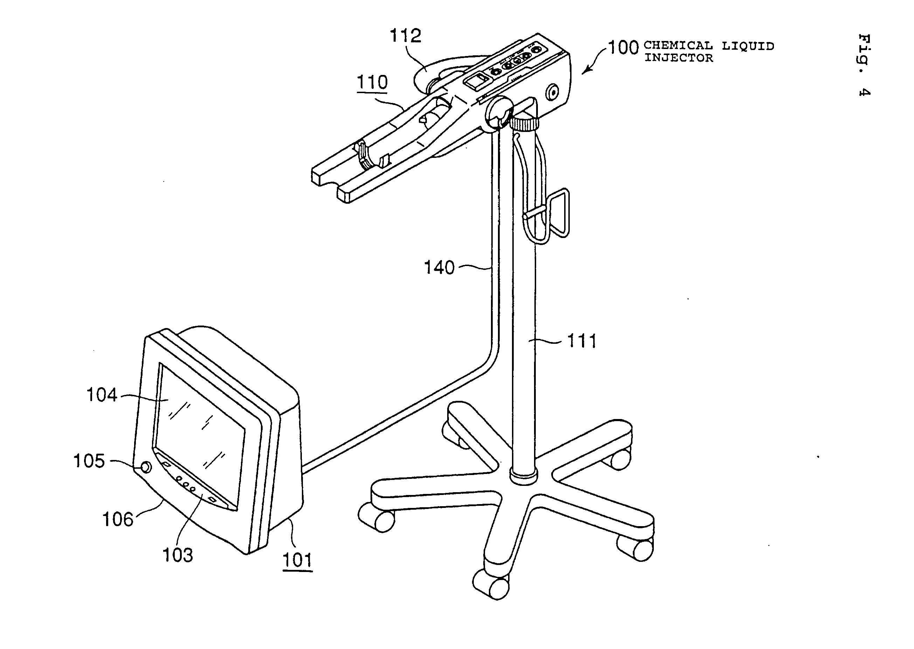

An embodiment of the present invention will hereinafter be described with reference to drawings. As shown in Fig. 4, chemical liquid injector 100 of the embodiment includes injection control unit 101 and injection head 110 constructed as separate components. Injection control unit 101 and injection head 110 serving as an injection operation unit are wire-connected through data communication member 140 serving as a data communication apparatus.

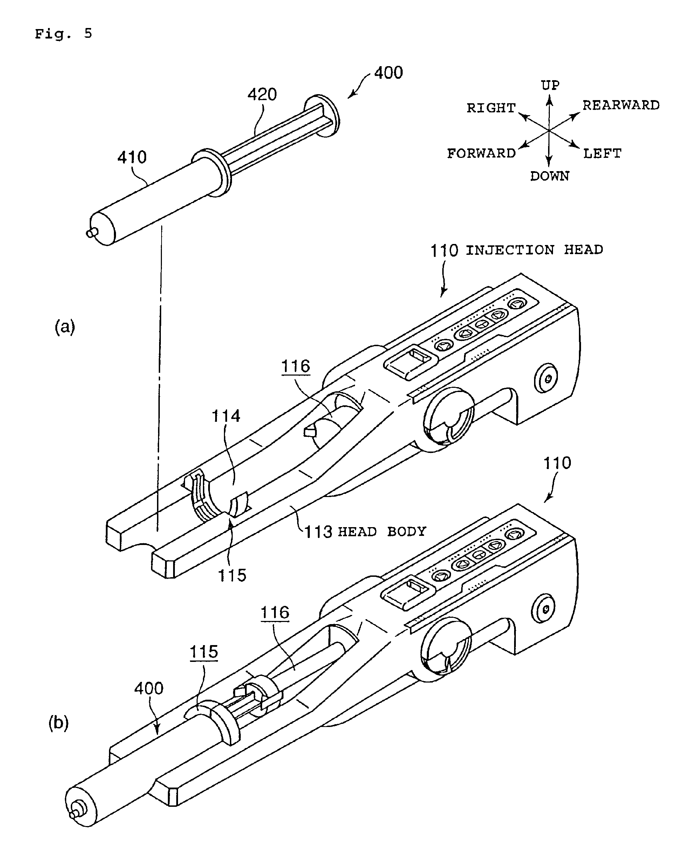

As shown in Fig. 4, injection control unit 101 has operation/touch panels 103, 104 serving as injection input means, speaker unit 105 and the like, all of which are disposed on the front face of unit housing 106. As shown in Fig. 5, liquid syringe 400 is exchangeably mounted on injection head 110 and is driven to inject a liquid such as a contrast medium for MR into a patient.

Liquid syringe 400 comprises cylinder member 410 and piston member 420 wherein piston member 420 is slidably inserted into cylinder member 410. Cylinder member 410 is filled with a liquid such as physiological saline or a contrast medium for MR. The liquid can be injected into the patient.

Injection head 110 is attached to the top end of caster stand 111 by movable arm 112. As shown in Fig. 5, head body 113 of injection head 110 has concave portion 114 formed as a semi-cylindrical groove in the upper surface for removably mounting liquid syringe 400. Cylinder holding mechanism 115 is formed in the forward portion of concave portion 114 for removably holding cylinder member 410 of liquid syringe 400. Piston driving mechanism 116 as a liquid injection means is arranged in the rearward portion of concave portion 114 for holding and sliding piston member 420.

Cylinder holding mechanism 115 is openable and closable to removably hold cylinder member 410. Piston driving mechanism 116 has ultrasonic motor 118 as a driving source which is free from generation of magnetic field even in operation, and slides piston member 420 through a screw mechanism (not shown) or the like.

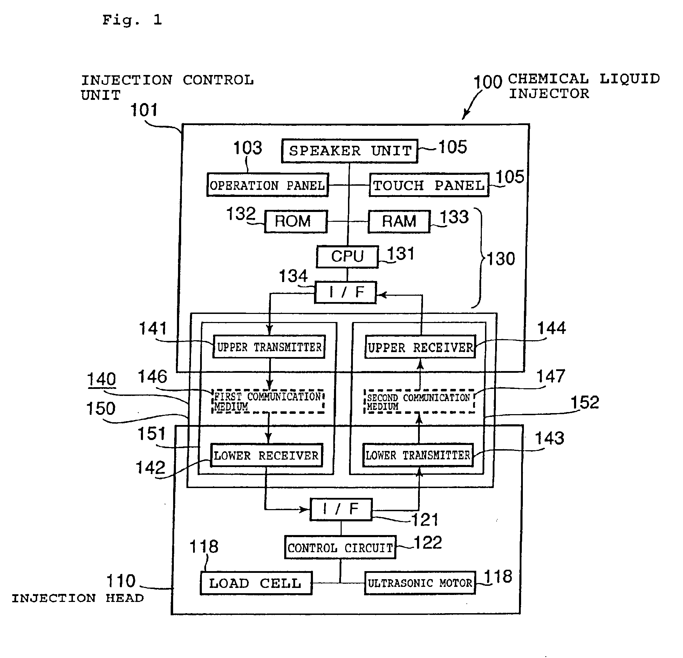

As shown in Fig. 1, injection head 110 also has load cell 119, communication I/F (Interface) 121 corresponding to an injection receiving means, and head control circuit 122. Ultrasonic motor 118, load cell 119, and communication I/F 121 are connected to head control circuit 122. On the other hand, injection control unit 101 has computer unit 130 which is formed of a one-chip microcomputer provided with hardware such as CPU 131, ROM 132, RAM 133, and communication I/F 134 corresponding to an injection transmitting means.

ROM 132, RAM 133, communication I/F 134, operation panel 103, touch panel 104, speaker unit 105 and the like are connected to CPU 131. Communication I/F 134 of injection control unit 101 is connected to communication I/F 121 of injection head 110 through data communication member 140.

Computer unit 130 produces injection data in response to data entered through operation panel 103 or touch panel 104. The injection data is transmitted to head control circuit 122 of injection head 110 though data communication member 140. Head control circuit 122 controls the operation of ultrasonic motor 118 in accordance with the received injection data. As a result, the injection of the liquid is controlled in accordance with the entry on operation panel 103 or the like.

Load cell 119 detects the pressure of the liquid injected into the patient from liquid syringe 400 by piston driving mechanism 116. The detection result is transmitted by head control circuit 122 to computer unit 130 of injection control unit 101 through data communication member 140.

Computer unit 130 outputs the received injection pressure with display on touch panel 104. In this manner, the injection pressure for the liquid is presented to the operator on touch panel 104 of injection control unit 101. Since computer unit 130 incorporates the received injection pressure into the injection data and changes the injection data as required, the injection of the liquid at an excessive pressure is prevented.

Data communication member 140 intervening in data communication between injection control unit 101 and injection head 110 has tube member 150, upper transmitter 141, lower receiver 142, lower transmitter 143, upper receiver 144, first communication medium 146, second communication medium 147 and the like.

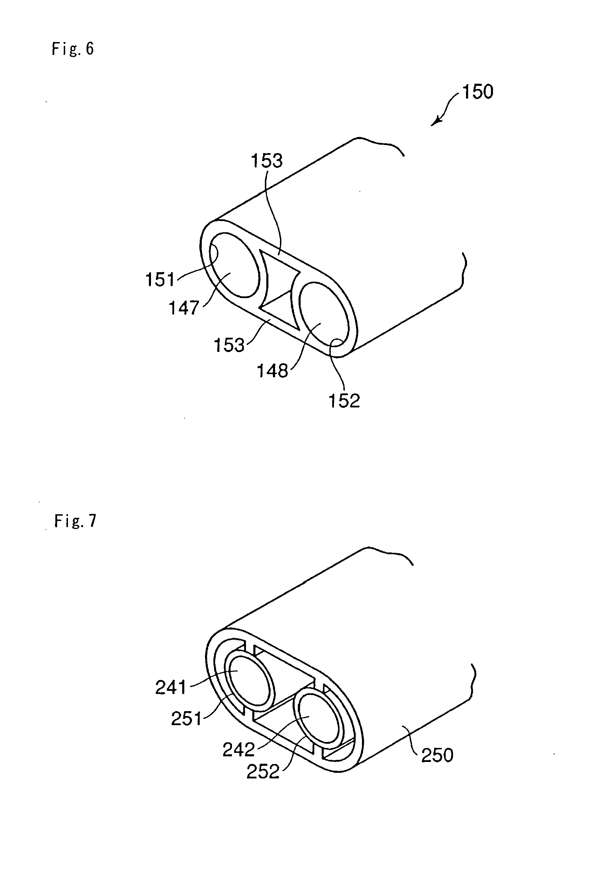

Tube member 150 is elongated and flexible as shown in Fig. 4 and is formed of resin or the like. As shown in Fig. 6, tube member 150 has first tube chamber 151 serving as a first sealing case and second tube chamber 152 serving as a second sealing case. First tube chamber 151 and second tube chamber 152 are formed as elongated cylinders and arranged in parallel such that they are separated by a pair of connecting portions 153 serving as a case paralleling means.

First tube chamber 151 has upper transmitter 141 at one end and lower receiver 142 at the other end. First communication medium 146 formed of fluid such as silicone oil is enclosed in first tube chamber 151. Second tube chamber 152 has lower transmitter 143 at one end and upper receiver 144 at the other end. Second communication medium 147 formed of fluid such as silicone oil is enclosed in second tube chamber 152.

Upper transmitter 141 converts an electric signal transmitted from communication I/F 134 of injection control unit 101 into an ultrasonic signal for transmission. Lower receiver 142 converts the received ultrasonic signal into an electric signal and transmits the electric signal to communication I/F 121 of injection head 110. Lower transmitter 143 converts an electric signal transmitted from communication I/F 121 of injection head 110 into an ultrasonic signal for transmission. Upper receiver 144 converts the received ultrasonic signal into an electric signal and transmits the electric signal to communication I/F 134 of injection control unit 101.

As described above, the ultrasonic signal is transported from upper transmitter 141 to lower receiver 142 through first communication medium 146, while the ultrasonic signal is transported from lower transmitter 143 to upper receiver 144 through second communication medium 147. Since first/second tube chambers 151 and 152 containing first/second communication media 146/147 are arranged in parallel such that they are separated by connecting portion 153, the ultrasonic signal transported through one of first/second communication media 146, 147 is unlikely to pass to the other of the media.

In chemical liquid injector 100 of the embodiment, respective components are formed of nonmagnetic material, and the portions which cannot be formed of nonmagnetic material are magnetically shielded. For example, ultrasonic motor 118 is formed of nonmagnetic metal such as phosphor bronze alloy (Cu + Sn +P), titanium alloy (Ti-6Al-4V), and magnesium alloy (Mg + Al +Zn). Head body 113 or the like is formed of nonmagnetic resin.

Thus, upper/lower transmitters 141, 143 and upper/lower receivers 144, 142 of data communication member 140 are also formed of nonmagnetic metal or resin. It goes without saying that tube member 150 and first/second communication media 146, 147 are also formed of nonmagnetic material such as resin and silicone oil.

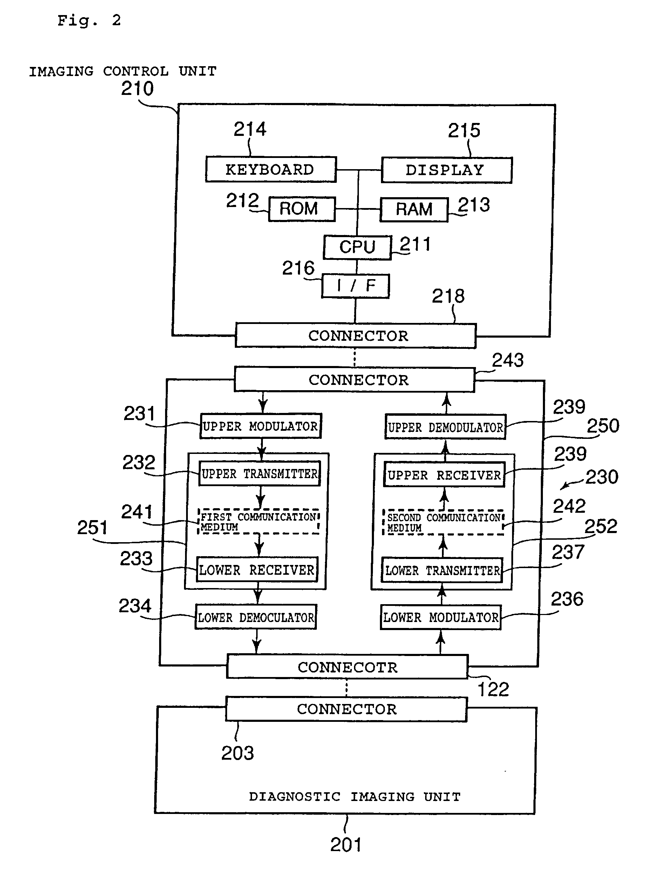

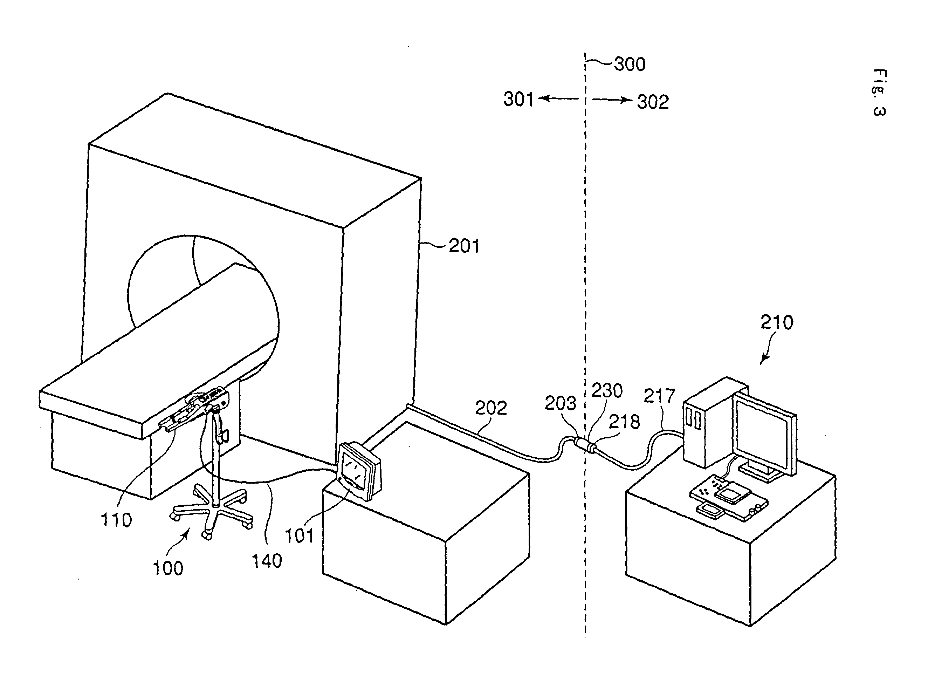

Chemical liquid injector 100 of the embodiment injects the contrast medium or physiological saline as the liquid into the patient whose diagnostic image is to be taken by MRI apparatus 200 as described above. As shown in Figs. 2 and 3, MRI apparatus 200 of the embodiment has diagnostic imaging unit 201 serving as an imaging operation mechanism and imaging control unit 210. Diagnostic imaging unit 201 captures diagnostic images of the patient, while imaging control unit 201 controls the operation of imaging diagnostic unit 201.

As shown in Fig. 2, imaging control unit 210 of MRI apparatus 200 has hardware such as CPU 211, ROM 212, RAM 213, keyboard 214 serving as an imaging entry means, display 215 serving as an image display means, and communication I/F 216 serving as an imaging transmitting means and an image receiving means. The abovementioned components 212 to 216 are connected to CPU 211.

Communication cable 202 is fixedly connected to diagnostic imaging unit 201. Communication cable 217 is fixedly connected to communication I/F 216 of imaging control unit 210. Communication cables 202, 217 have connectors 203, 218 at their ends. Connectors 203, 218 are removably connected.

As shown in Fig. 3, in MRI apparatus 200 of the embodiment, diagnostic imaging unit 201 is placed in treatment room 301 separated from operation room 302 by wall 300, and imaging control unit 210 is placed in operation room 302. Connectors 203, 218 are connected to data communication unit 230 serving as a data communication apparatus buried in wall 300.

Data communication unit 230 intervening in data communication between injection control unit 101 and injection head 110 as described above has unit body 250 serving as case paralleling means, upper modulator 231, upper transmitter 232, lower receiver 233, lower demodulator 234, lower modulator 236, lower transmitter 237, upper receiver 238, upper demodulator 239, first communication medium 241, second communication medium 242, upper connector 243, and lower connector 244.

As shown in Fig. 7, unit body 250 has a flat cylindrical shape formed of hard resin or the like. First sealing case 251 and second sealing case 252 are placed in unit body 250. First sealing case 251 and second sealing case 252 are formed in cylindrical shape and arranged in parallel such that they are separately supported each other integral with unit body 250.

Upper connector 243 is mounted on one end of unit body 250. Lower connector 244 is mounted on the other end thereof. Connector 218 of imaging control unit 210 is removably connected to upper connector 243, while connector 203 of diagnostic imaging unit 201 is removably connected to lower connector 244.

Connected to upper connector 243 are upper transmitter 232 via upper modulator 231 and upper receiver 238 via upper demodulator 239. Connected to lower connector 244 are lower receiver 233 via lower demodulator 234 and lower transmitter 237 via lower modulator 236.

First sealing case 251 has upper transmitter 232 at one end and lower receiver 233 at the other end. First communication medium 241 formed of fluid such as silicone oil is enclosed in first sealing case 251. Second sealing case 252 has lower transmitter 237 at one end and upper receiver 238 at the other end. Second communication medium 242 formed of fluid such as silicone oil is enclosed in second sealing case 252.

Upper modulator 231 converts an electric signal parallel transmitted from imaging control unit 210 into a serial signal and transmits the serial signal to upper transmitter 232. Upper transmitter 232 converts the serially transmitted electric signal into an ultrasonic signal for transmission. Lower receiver 233 converts the received ultrasonic signal into an electric signal and serially transmits the electric signal to lower demodulator 234. Lower demodulator 234 converts the serially transmitted electric signal into a parallel signal and transmits the parallel signal to diagnostic imaging unit 201.

Lower modulator 236 converts an electric signal parallel transmitted from diagnostic imaging unit 201 into a serial signal and transmits the serial signal to lower transmitter 237. Lower transmitter 237 converts the serially transmitted electric signal into an ultrasonic signal for transmission. Upper receiver 238 converts the received ultrasonic signal into an electric signal and serially transmits the electric signal to upper demodulator 239. Upper demodulator 239 converts the serially transmitted electric signal into a parallel signal and transmits the parallel signal to imaging control unit 210.

As described above, first communication medium 241 transports the ultrasonic signal from upper transmitter 232 to lower receiver 233, while second communication medium 242 transports the ultrasonic signal from lower transmitter 237 to upper receiver 238. Since first/second sealing cases 251, 252 containing first/second communication media 241, 242 are arranged in parallel such that they are separated each other by connecting portion 153, the ultrasonic signal transported through one of first/second communication media 241, 242 is unlikely to pass to the other of the media.

### (Operation of the Embodiment)

In the abovementioned configuration, when chemical liquid injector 100 and MRI apparatus 200 of the embodiment are used, chemical liquid injector 100 and diagnostic imaging unit 201 are placed in treatment room 301 as shown in Fig. 3. Imaging control unit 210 is placed in operation room 302. Communication cables 202, 217 of diagnostic imaging unit 201 and imaging control unit 210 are connected to data communication unit 230 buried in wall 300.

Liquid syringe 400 filled with the liquid such as contrast medium for MR is provided together with an extension tube (not shown). Liquid syringe 400 is connected to a patient (not shown) in diagnostic imaging unit 201 through the extension tube. Liquid syringe 400 is mounted on injection head 110 of chemical liquid injector 100.

In this state, two operators (not shown) take the places such that one of them is in treatment room 301 for operation of chemical liquid injector 100 and the other is in operation room 302 for operation of diagnostic imaging apparatus 200. One of the operators manually operates chemical liquid injector 100 in treatment room 301 to inject the contrast medium into the patient from liquid syringe 400, while the other manually operates imaging control unit 210 in operation room 302 to cause diagnostic imaging unit 201 in treatment room 301 to capture diagnostic images of the patient.

More particularly, when the operator in treatment room 301 manually operates and makes entry into operation panel 103 or touch panel 104 of injection control unit 101, computer unit 130 transmits the injection data to upper transmitter 141 of data communication member 140 from communication I/F 134 in response to the entered data.

Then, upper transmitter 141 converts the injection data provided as the electric signal into an ultrasonic signal for transmission. The ultrasonic signal is transported to lower receiver 142 through first communication medium 146. Lower receiver 142 converts the received ultrasonic signal into an electric signal and transmits the electric signal to communication I/F 121 of injection head 110. In this manner, the injection data is transmitted from injection control unit 101 to injection head 110.

In injection head 110, head control circuit 122 controls the operation of ultrasonic motor 118 in accordance with the injection data. Thus, the contrast medium is injected from liquid syringe 400 into the patient based on the entry operation. The pressure of the injected contrast medium is detected by load cell 119, and the detected injection pressure is transmitted as an electric signal by head control circuit 122 from communication I/F 121 to lower transmitter 143 of data communication member 140.

Then, lower transmitter 143 converts the electric signal representing the injection pressure into an ultrasonic signal for transmission. The ultrasonic signal is transmitted to upper receiver 144 through second communication medium 147. Upper receiver 144 converts the received ultrasonic signal into an electric signal and transmits the electric signal to communication I/F 134 of injection control unit 101. In this manner, the injection pressure is transmitted to injection control unit 101 from injection head 110.

In injection control unit 101, computer unit 130 outputs the received injection pressure with display on touch panel 104, thereby presenting the injection pressure of the contrast medium to the operator on touch panel 104. Since computer unit 130 incorporates the received injection pressure into the injection data and changes the injection data as required, the injection of the liquid at an excessive pressure is prevented.

On the other hand, the operator in operation room 302 manually operates imaging control unit 210 of MRI apparatus 200 to cause diagnostic imaging unit 201 to capture diagnostic images of the patient when the contrast medium is injected into the patient from chemical liquid injector 100 as described above. When the operator in operation room 302 manually operates keyboard 214 of imaging control unit 210, CPU 211 produces imaging data in response to the entered data, and the imaging data is parallel transmitted to data communication unit 210 from communication I/F 216.

In data communication unit 210, upper modulator 231 converts the parallel transmitted imaging data into a serial signal, and upper transmitter 232 converts the serial electric signal into an ultrasonic signal for transmission. The ultrasonic signal is transported to lower receiver 233 through first communication medium 241. Lower receiver 233 converts the received ultrasonic signal into an electric signal and serially transmits the electric signal to lower demodulator 234.

Then, lower demodulator 234 converts the serially transmitted electric signal into a parallel signal and transmits the parallel signal to diagnostic imaging unit 201. In this manner, the imaging data is transmitted from imaging control unit 210 to diagnostic imaging unit 201. Diagnostic imaging unit 201 captures diagnostic images from the patient in accordance with the imaging data, and parallel transmits the diagnostic images as electric signals to data communication unit 230.

In data communication unit 230, lower modulator 236 converts the parallel transmitted electric signal representing the diagnostic image into a serial signal. Lower transmitter 237 converts the serial signal into an ultrasonic signal for transmission. The ultrasonic signal is transported to upper demodulator 239 through second communication medium 242. Upper receiver 238 converts the received ultrasonic signal into an electric signal and serially transmits the electric signal to upper demodulator 239.

Then, upper demodulator 239 converts the serially transmitted electric signal to a parallel signal and transmits the parallel signal to imaging control unit 210. In this manner, the diagnostic image is transmitted from diagnostic imaging unit 201 to imaging control unit 210. Imaging control unit 210 outputs the received diagnostic image on display 215 to allow the operator to observe the captured diagnostic image.

### (Effect of the Embodiment)

In chemical liquid injector 100 of the embodiment, the respective components of injection control unit 101 and injection head 110 are made of nonmagnetic material, and the portions which cannot be made of nonmagnetic material are magnetically shielded. In addition, injection control unit 101 and injection head 110 are connected to each other through elongated and flexible data communication member 140 which intervenes in the data communication between injection control unit 101 and injection head 110. Data communication member 140 transmits the electric signal between injection control unit 101 and injection head 110 as the ultrasonic signal.

Thus, even when chemical liquid injector 100 of the embodiment is used near MRI apparatus 200, the magnetic field of MRI apparatus 200 is not affected. On the other hand, the magnetic field produced by MRI apparatus 200 does not affect the ultrasonic signal transported through data communication member 140, so that chemical liquid injector 100 of the embodiment does not malfuntion even when it is used near MRI apparatus 200.

In data communication member 140, first/second communication media 146, 147 formed of liquid such as silicone oil are enclosed in first/second tube chambers 151, 152 of tube member 150, which enables favorable transport of the ultrasonic signal with the simple structure. Since the entire structure of data communication member 140 is flexibly formed, injection control unit 101 and injection head 110 can be arranged freely.

Injection control unit 101 and injection head 110 communicate data with each other bidirectionally. As shown in Fig. 6, since first/second tube chambers 151, 152 of data communication member 140 are arranged in parallel such that they are separated from each other, interference of the ultrasonic signals transmitted in both directions is prevented.

As shown in Fig. 7, data communication unit 230 intervening in the data communication between imaging control unit 210 and diagnostic imaging unit 201 of MRI apparatus 200 includes first/second sealing cases 251, 252 arranged in parallel such that they are separated from each other. This also can prevent interference of the ultrasonic signals transmitted in both directions.

MRI apparatus 200 of the embodiment realizes the data communication between imaging control unit 210 and diagnostic imaging unit 201 by using the parallel signal. Since data communication unit 230 converts the parallel signal into the serial signal before conversion into the ultrasonic signal, the parallel electric signal can be communicated after the conversion into the serial ultrasonic signal.

Data communication member/unit 140, 230 convert the electric signal into the ultrasonic signal for transmission as described above, which can reliably regulate the transmission direction of the electric signal. Thus, according to data communication member/unit 140, 230 of the embodiment, even when noise is introduced into lower/upper receivers 142, 144, 233, 238, the noise is not passed to upper/lower transmitters 141, 143, 232, 237. The malfunction of chemical liquid injector 100 and MRI apparatus 200 can be prevented reliably.

### (Modifications of the Embodiment)

The present invention is not in any way limited to the abovementioned embodiment, but various changes and modifications may be made therein without departing from the scope of the invention. For example, in the above embodiment, standalone MRI apparatus 200 and chemical liquid injector 100 operate individually. However, MRI apparatus 200 and chemical liquid injector 100 may perform various operations in association with each other through data communication or the like.

In this case, a data communication apparatus of the similar structure as that of data communication member 140 can be used to connect chemical liquid injector 100 with MRI apparatus 200 (not shown) to perform data communication without producing magnetic wave noise from chemical liquid injector 100 and MRI apparatus 200 and thus with no influence of electromagnetic wave noise.

In the above embodiment, data communication member 140 having upper/lower transmitters 141, 143 and upper/lower receivers 144, 142 is used to perform the communication between injection control unit 101 and injection head 110 of chemical liquid injector 100. Alternatively, it is possible to use a data communication member (not shown) having only upper transmitter 141 and lower receiver 142 to perform data communication bidirectionally from injection control unit 101 to injection head 110.

In the above embodiment, first/second communication media 146, 147, 241, 242 formed of fluid are enclosed in first/second tube chambers 151, 152 of tube member 150 and first/second sealing cases 251, 252. Such first/second communication media may be formed of lumps of resin (not shown), for example.

In the above embodiment, MRI apparatus 200 is used as the diagnostic imaging apparatus and the contrast medium for MR is injected as the liquid by chemical liquid injector 100. Alternatively, a CT scanner or a PET apparatus or an ultrasonic diagnostic apparatus may be used as the imaging diagnostic apparatus and a contrast medium intended therefor may be injected by the chemical liquid injector.

In the abovementioned embodiment, CPU 131 operates according to a computer program stored on RAM 133 or the like to logically realize various means as various functions of chemical liquid injector 100. Each of the various means may be formed as specific hardware, or some of them may be stored as software on RAM 133, while others may be formed as hardware.

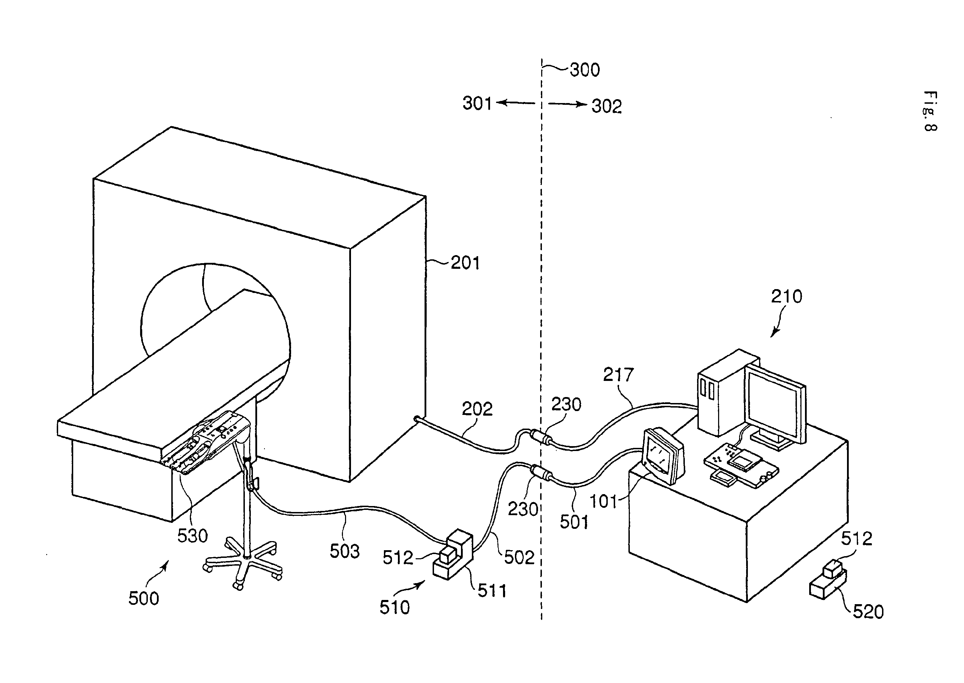

The above embodiment has shown chemical liquid injector 100 in which only one liquid syringe 400 is mounted on injection head 110. For example, as shown in Fig. 8, it is possible to realize chemical liquid injector 500 in which two liquid syringes 200 for a contrast medium and physiological saline and the like are mounted on injection head 530 or a chemical liquid injector (not shown) in which three or more liquid syringes 200 are mounted on an injection head.

In the above embodiment, to simplify the description, injection control unit 101 and injection head 110 of chemical liquid injector 100 are connected directly through data communication member 140, and all of them are placed in treatment room 301 together with imaging diagnostic unit 201 of MRI apparatus 200.

In chemical liquid injector 500 actually used with MRI apparatus 200, however, injection head 110 is placed in treatment room 301 and injection control unit 101 is generally placed in operation room 302. A structure in such case will be described in brief with reference to Fig. 8.

In chemical liquid injector 500, communication cable 501 which is not magnetically shielded is connected into injection control unit 101. Communication cable 501 is connected to one end of second data communication unit 230 buried in wall 300. Power unit 510 is connected to the other end of data communication unit 230 via magnetically shielded communication cable 502. Injection head 110 is connected to power unit 510 via magnetically shielded communication cable 503.

Power unit 501 has unit body 511 and secondary battery 512 which is removably mounted on unit body 511. Charging unit 520 is placed in operation room 302. Secondary battery 512 is also removably mounted on charging unit 520. Power unit 510 and secondary battery 512 are formed of nonmagnetic metal such as phosphor bronze alloy (Cu + Sn +P), titanium alloy (Ti-6Al-4V), and magnesium alloy (Mg + Al +Zn), or nonmagnetic resin.

In chemical liquid injector 500 as described above, injection control unit 101 in operation room 302 communicates data with injection head 110 in treatment room 301 via communication cable 501, data communication unit 230, communication cable 502, power unit 510, and then communication cable 503 in this order.

Power unit 510 outputs various types of data entered from injection control unit 101 to injection head 110 and supplies the power of secondary battery 512 to injection head 110 through communication cable 503. Thus, any commercial power does not need to be placed in operation room 301, thereby preventing disturbance of the magnetic field of imaging diagnostic unit 201 more satisfactorily.

A commercial power (not shown) is placed in operation room 210. Injection control unit 101 is supplied with power from the commercial power. Charging unit 520 is connected to the commercial power in operation room 302 and charges secondary battery 512.

## Claims

1. A data communication apparatus for intervening in communication of an electric signal from an upper electronic device to a lower electronic device, comprising:
an ultrasonic transmitter converting an electric signal transmitted from an upper electronic device into an ultrasonic signal and transmitting the ultrasonic signal;
an ultrasonic receiver receiving and converting the ultrasonic signal transmitted from the ultrasonic transmitter into an electric signal and transmitting the electric signal to a lower electronic device; and
an ultrasonic communication medium transporting the ultrasonic signal, the ultrasonic transmitter and the ultrasonic receiver being placed in the ultrasonic communication medium such that the ultrasonic transmitter and the ultrasonic receiver are separated from each other.

2. The data communication apparatus according to claim 1, wherein the ultrasonic communication medium is a fluid, and
the data communication apparatus further comprising a medium sealing case in which the ultrasonic communication medium is contained.

3. The data communication apparatus according to claim 1, wherein the ultrasonic communication medium is flexible and formed in elongated shape, and
the ultrasonic transmitter is placed at one end of the ultrasonic communication medium and the ultrasonic receiver is placed at the other end of the ultrasonic communication medium.

4. The data communication apparatus according to claim 2, wherein the medium sealing case is flexible and formed in elongated tube shape, and
the ultrasonic transmitter is placed at one end of the medium sealing case and the ultrasonic receiver is placed at the other end of the medium sealing case.

5. The data communication apparatus according to any one of claims 1 to 4, further comprising:
an upper modulator converting an electric signal parallel transmitted from the upper electronic device into a serial signal and transmitting the serial signal to the ultrasonic transmitter; and
a lower demodulator converting the electric signal serially transmitted from the ultrasonic receiver into a parallel signal and transmitting the parallel signal to the lower electronic device.

6. A data communication apparatus for intervening in communication of an electric signal between an upper electronic device and a lower electronic device, comprising:
an upper transmitter converting an electric signal transmitted from an upper electronic device into an ultrasonic signal and transmitting the ultrasonic signal;
a lower receiver receiving and converting the ultrasonic signal transmitted from the upper transmitter into an electric signal and transmitting the electric signal to the lower electronic device;
a lower transmitter converting an electric signal transmitted from a lower electronic device into an ultrasonic signal and transmitting the ultrasonic signal;
an upper receiver receiving and converting the ultrasonic signal transmitted from the lower transmitter into an electric signal and transmitting the electric signal to the upper electronic device;
a first communication medium transporting the ultrasonic signal, the upper transmitter and the lower receiver being placed in the first communication medium such that the upper transmitter and the lower receiver are separated from each other; and
a second communication medium transporting the ultrasonic signal, the lower transmitter and the upper receiver being placed in the second communication medium such that the lower transmitter and the upper receiver are separated from each other.

7. The data communication apparatus according to claim 6,
wherein each of the first communication medium and the second communication medium is a fluid,
the data communication apparatus further comprising:
a first sealing case in which the first communication medium is contained; and
a second sealing case in which the second communication medium is contained.

8. The data communication apparatus according to claim 6,
wherein each of the first communication medium and the second communication medium is flexible and formed in elongated shape,
the upper transmitter is placed at one end of the first communication medium and the lower receiver is placed at the other end of the first communication medium,
the lower transmitter is placed at one end of the second communication medium and the upper receiver is placed at the other end of the second communication medium, and
the data communication apparatus further comprising medium paralleling means for arranging the first communication medium and the second communication medium in parallel such that the ultrasonic signal transmitted through one of the media does not pass to the other.

9. The data communication apparatus according to claim 7, wherein the first sealing case and the second sealing case is flexible and formed in elongated tube shape,
the upper transmitter is placed at one end of the first sealing case and the lower receiver is placed at the other end of the first sealing case,
the lower transmitter is placed at one end of the second sealing case and the upper receiver is placed at the other end of the second sealing case, and
the data communication apparatus further comprising case paralleling means for arranging the first sealing case and the second sealing case in parallel such that the ultrasonic signal transmitted in one of the cases does not pass to the other.

10. The data communication apparatus according to any one of claims 6 to 9, further comprising:
an upper modulator converting an electric signal parallel transmitted from the upper electronic device into a serial signal and transmitting the serial signal to the upper transmitter;
a lower demodulator converting the electric signal serially transmitted from the lower receiver into a parallel signal and transmitting the parallel signal to the lower electronic device;
a lower modulator converting an electric signal parallel transmitted from the lower electronic device into a serial signal and transmitting the serial signal to the lower transmitter; and
an upper demodulator converting the electric signal serially transmitted from the upper receiver into a parallel signal and transmitting the parallel signal to the upper electronic device.

11. A chemical liquid injector for injecting a liquid into a patient whose diagnostic image is to be taken by an diagnostic imaging unit of a diagnostic imaging apparatus, comprising:
a data communication apparatus according to any one of claims 3, 4, 8, and 9, having an injection control unit to which injection data for controlling injection of the liquid is entered, an injection operation unit performing injection of the liquid in accordance with the injection data, the injection control unit being connected as the upper electronic device to the data communication apparatus, and the injection operation unit being connected as the lower electronic device to the data communication apparatus,
wherein the injection control unit comprises injection input means for receiving the entry of the injection data and injection transmitting means for transmitting the entered injection data to the upper transmitter of the data communication apparatus, and
the injection operation unit comprises injection receiving means for receiving the injection data transmitted from the lower receiver of the data communication apparatus and liquid injection means for injecting the liquid into the patient in accordance with the received injection data.

12. An diagnostic imaging apparatus comprising:
a diagnostic imaging unit capturing a diagnostic image of a patient and outputting the diagnostic image to the outside;
an imaging control unit remotely controlling the diagnostic imaging unit and receiving and outputting the diagnostic image as display; and
the data communication apparatus according to any one of claims 6 to 10, the imaging control unit being connected as the upper electronic device to the data communication apparatus and the diagnostic imaging unit being connected as the lower electronic device to the data communication apparatus,
wherein the imaging control unit comprises imaging input means for receiving the entry of imaging data for controlling imaging operation, imaging transmitting means for transmitting the entered imaging data to the upper transmitter of the data communication apparatus, image receiving means for receiving the diagnostic image transmitted from the upper receiver of the data communication apparatus, and image display means for displaying the received diagnostic image, and
the diagnostic imaging unit comprises imaging receiving means for receiving the imaging data transmitted from the lower receiver of the data communication apparatus, imaging means for imaging the diagnostic image of the patient in accordance with the received imaging data, and image transmitting means for transmitting the diagnostic image to the lower transmitter of the data communication apparatus.

13. The diagnostic imaging apparatus according to claim 12, wherein the diagnostic imaging unit and the imaging control unit are placed in different rooms separated by a wall, and
the data communication apparatus is buried in the wall.
